# EUROPEAN PATENT APPLICATION

(11) **EP 3 095 478 A1**
(43) Date of publication of application: **23.11.2016**
(21) Application number: 15305745.0
(22) Date of filing: 19.05.2015
(51) Int. Cl.: A61M 16/06

(54) **RESPIRATORY MASK WITH IMPROVED CUSHION**

(71) Applicant: Air Liquide Medical Systems, 92160 Antony (FR)
(72) Inventor: Alberici, Luca, 25030 RONCADELLE (BS) (IT); Masserdotti, Fulvio, 25075 BRESCIA (IT); Sandoni, Giuseppe, 25124 BRESCIA (IT)
(74) Representative: Pittis, Olivier

(57) **Abstract**

The invention concerns a respiratory mask (20) with an improved single-lip cushion (1) that is useable for treatment of a respiratory disorder or condition in non-invasive positive pressure ventilation or in a nasal continuous positive airway pressure therapy of sleep disordered breathing conditions, such as obstructive sleep apnea. The single-lip cushion (1) comprises a unique membrane (3) comprising a cradle-like (7) structure arranged in the nasal bridge region so as to better accommodate the nasal bridge area of the user thereby ensuring improved comfort and gas sealing.

## Description

The invention concerns a "single-lip" or single membrane cushion for respiratory mask, in particular for a facial mask, for adult or pediatric uses in the treatment of respiratory conditions or diseases affecting adults, children, toddlers, infants or newborns.

Respiratory masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)... The flow of breathable gas delivered by the mask can be, for instance, air or air enriched with oxygen.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face. This face-contacting cushion is usually made of soft, resilient elastomeric material, such as soft silicone or similar material.

Masks additionally may have a forehead support and a headgear for correctly positioning, maintaining and/or securing the mask on the head of the patient wearing the mask.

Examples of respiratory masks are given by documents : EP-A-462701, EP-A-874667, EP-A-1972357 and WO-A-00/57942.

However, the current masks are not ideal, especially when patients have to wear them overnight, as many of them are difficult to be well-positioned on the face of the patients and well-maintained thereafter, without gas leaks and discomfort for the patients.

In particular, gas tightness is a recurrent problem with respiratory masks.

Gas leaks often appear between the cushion of the mask and the patient's face, especially in the nasal bridge region of the cushion. Indeed, as the nasal bridge area of the nose varies from one person to another, it is often difficult to achieve an efficient gas tight seal between the cushion of the mask and the nasal bridge area of several patients, when considering a given mask.

For trying to overcome this problem, document EP-A-1741461 proposes a "double-lip" cushion for a nasal mask comprising a frame supporting an inner rim surrounding the patient's nose, also called "inner membrane" or "inner lip", and an outer membrane, also called "outer lip", spaced from the rim. The outer membrane and the inner rim have a same general shape so that the outer membrane overlies the inner rim. The outer membrane is made of resilient material so as to be flexible and come into contact with the rim, when the cushion is applied against the face of the patient, thereby ensuring gas sealing. For accommodating the bridge of the nose of the patient, the inner rim and the outer membrane both comprise a notch, i.e. a kind of cut or removal of material, situated in the nasal bridge regions of said rim and membrane.

However, if such a structure may work with a "double-lip" cushion comprising an inner rim and a superimposed outer membrane, as both cooperate for forming the gas seal, it is not suitable when the cushion is a "single-lip" one that does not comprises any inner rim, but only a single outer membrane that ensures alone the gas tightness.

Indeed, in that case, providing a notch in the nasal bridge region of the single membrane of a "single-lip" cushion leads to sealing failures and inevitable gas leaks in said region, and involves a discomfort for the patient, especially when the mask is worn for hours, such as overnight.

In this context, the problem to be solved is to propose an improved respiratory "single-lip" cushion, especially a cushion for a facial mask, that ensures efficient tightness, i.e. gas seal, preventing or minimizing the escape of gas, i.e. gas leaks, in the nasal bridge area of the nose of the user, without negatively impacting the comfort of use for the patient, despite the fact that the "single-lip" cushion comprises a unique or single membrane, i.e. no inner rim.

The solution of the present invention concerns a "single-lip" cushion for a respiratory mask, in particular a facial mask, comprising a frame having a generally-triangular shape and a single flexible membrane integrally fixed to the frame, and wherein the flexible membrane comprises a central aperture, i.e. opening, for receiving at least the nose of a user, i.e. a patient, and a nasal bridge region that comes into contact with the nasal bridge area of the patient's nose, when the cushion is positioned on the face of the user, characterized in that the nasal bridge region of the flexible membrane is pre-shaped so as to form a cradle that is inwardly-oriented and sized for accommodating at least the nasal bridge area of the nose of the user.

The single-lip cushion according to the present invention can further comprise one or more of the following additional features :
- the frame and the flexible membrane are made of a resilient material, preferably they are made of polymeric material, such as silicone.
- the flexible membrane further comprises an outer surface forming a convex sealing surface, i.e. a rounded surface, to the user's face, when the cushion is positioned on the face of a user when it is used. This ensures an efficient gas tightness while being in contact with the user's face, when the latter wears the mask.
- the inwardly-oriented cradle has a curved section.
- the inwardly-oriented cradle has a "U"-shape section.
- the inwardly-oriented cradle arranged in the flexible membrane is obtained by molding.
- the flexible membrane is fixed all along the frame and projects away from said frame.
- the flexible membrane is fixed along the perimeter of the frame.
- the flexible membrane comprises a free border delimiting the central aperture of said flexible membrane, i.e., the flexible membrane forms a flexible "skirt" around the central aperture.
- the flexible membrane and the frame are molded in one-piece.
- the frame further comprises a connecting border for fixing the cushion to a respiratory mask body.
- the thickness of the flexible membrane is less than the thickness of the frame.
- preferably, the thickness of the flexible membrane is of between 0,1 and 1 mm, typically of between 0,25 and 0,5 mm, and the thickness of the frame is of between 1,1 and 7 mm, typically of between 1,5 and 4,5 mm.
- the central aperture of the flexible membrane has a substantially triangular or trapezoidal shape.

The invention further concerns a respiratory mask comprising a single-lip cushion according to the present invention.

The respiratory mask according to the present invention can further comprise one or more of the following additional features:
- the single-lip cushion is fixed by means of the connecting border to a mask body delimiting a respiratory chamber comprising a gas inlet. The connecting border of the cushion cooperates with a complementary connecting structure of the mask so as to form a male/female connection for maintaining the cushion integral with the mask body. The male/female connection can comprise grooves, walls, abutments...
- the cushion is detachable from the mask body.
- the mask is a facial mask, wherein the cushion is sized to cover the nose and the mouth of the user when positioned on the user's face.
- the mask body comprises several arms.
- a headgear is fixed to the arms of the mask body.
- the mask body comprises a shell delimiting the respiratory chamber, preferably a rigid shell.
- the mask body comprises two lateral arms each projecting laterally on each side, i.e. right side and left side, of the mask body.
- the mask body comprises a holding arm projecting upwardly from the mask body.
- the two lateral arms and the holding arm are integral with mask body.
- the holding arm and of the two lateral arms have an elongated shape, for instance a band or ribbon shape. Of course, other shapes are possible.
- the mask body, the holding arm and of the two lateral arms are molded in one piece.
- the mask body, the holding arm and of the two lateral arms are made of a rigid material.
- the mask body, the holding arm and of the two lateral arms are made of a polymer material, such as polycarbonate (PC), polypropylene (PP) or nylon.
- the two lateral arms and the holding arm each comprises a free end, each free end comprising connecting structures for fixing thereto a headgear, especially the straps

of a headgear. The headgear is used for maintaining and securing the mask in a desired position on the head of the user, i.e. a patient. The straps of the headgear are typically made of polymer material or fabric material, or both.
- the gas inlet of the mask body has a diameter of between 1 and 3 cm.
- the gas inlet is located at the center of the front portion of the mask body.
- the holding arm has a length of about 2 and 10 cm.
- each lateral arms has a length of about 3 and 8 cm.
- the holding arm and of the two lateral arms are made of a flexible polymeric material so as to be slightly bendable toward the user's face under the forces applied by the headgear, when said headgear is adjusted to the morphology of the user's head.
- the headgear is fixed to the arms of the mask body by means of strap-fixing systems arranged at the free distal ends of said arms.
- the strap-fixing systems comprise slots and/or hooks.
- the holding arm that projects upwardly from the mask body comprises a forehead support.
- the forehead support can comprise one or several frontal pads that come into contact with the forehead of the user, when the mask is positioned on the user's face. The frontal pads act as bumpers and/or pillows that improve the comfort for the user that wears the mask.
- in a second embodiment, at least one strap of the headgear is arranged on the forehead support so as to come into contact with the forehead of the user, when the mask is positioned on the user's face. In that second embodiment, it is the strap itself that acts as a "pillow", i.e. a kind of smooth/soft structure, improving the comfort for the user that wears the mask.
- the central aperture of the cushion is adapted for receiving at least part of the user's nose, when the user wears the mask, preferably the nose and mouth of the user.
- the holding arm and the two lateral arms are configured to have curved-shapes.
- a hollow connector, preferably a curved rotatable connector, is fixed to the front side of the mask body and in fluid communication with the gas inlet of said mask body communicating with the respiratory chamber, thereby allowing a gas to circulate from the hollow connector to the respiratory chamber of the main body, or vice versa.
- the hollow curved connector is rotatable with respect to the main body.
- the hollow curved connector has general "L" shape.
- the upstream end of the tubular connecting piece is configured for connecting a gas line thereto.
- the cushion has a general triangular or saddle shape.
- the cushion is a facial cushion and comprises an upper region or "nasal bridge region", a lower region or "chin region" and two lateral nose regions or "cheek regions" linking the nasal bridge region to the chin region.

More precisely :
- the lower region or chin region is the area comprised between the mouth (i.e. lower lip of mouth) and the chin of the user;
- the upper region or nasal bridge region is the area of the nose approximately located at the junction of bone and cartilage, or above, i.e. on the nose bone ; and
- the two lateral nose regions or cheek regions are those located on each side of the nose.

The invention also concerns a medical ventilation assembly comprising a gas source, such as a medical ventilator, and a respiratory mask according to the present invention. Preferably, the gas source or gas delivery device is connected to the respiratory mask by means of a (or several) gas line or conduct, such as a flexible hose, preferably connected to the tubular connecting piece fixed to the hollow connector that is fixed to the mask body of the respiratory mask.

An embodiment of a single-lip cushion according to the present invention and a respiratory mask, especially a facial mask, comprising such a single-lip cushion are shown in the enclosed Figures, among which:
- Figures 1-3 represent different views of the single-lip cushion according to the present invention,
- Figure 4 is a sectional view of the cushion of Figures 1-3,
- Figures 5-6 are schematic views of the cushion of Figure 4 positioned on the face of a user, and
- Figures 7-8 show an embodiment of a facial mask equipped with the single-lip cushion of Figures 1-3.

The present invention proposes a single-lip cushion 1 for a respiratory mask as well as a respiratory mask 20 comprising such a single-lip cushion 1, as shown in the enclosed Figures that illustrate one embodiment of such a facial mask 20 with such a single-lip cushion 1 according to the present invention.

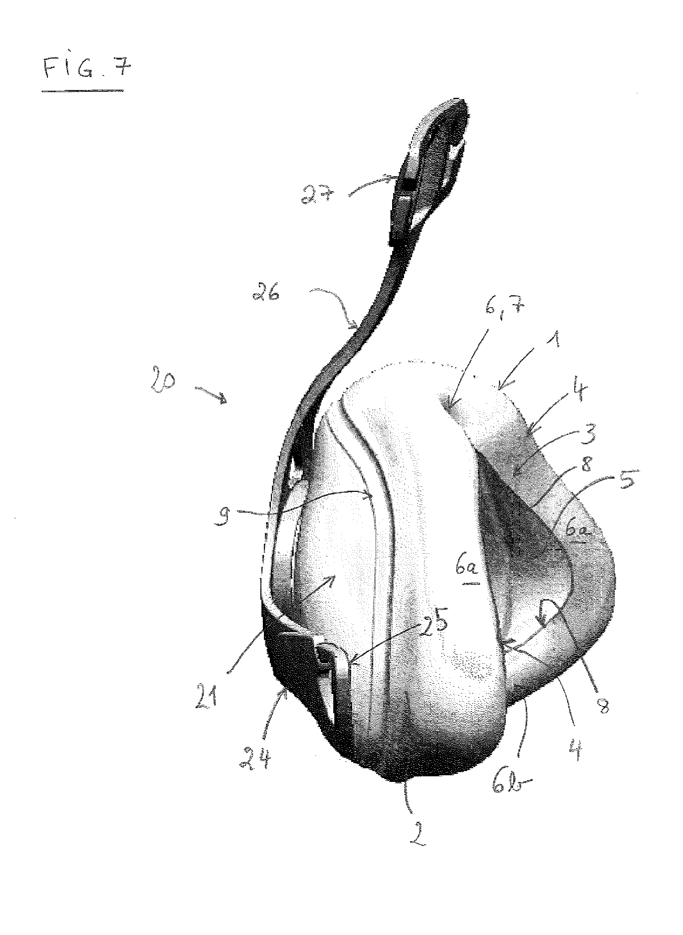

Generally speaking, as illustrated in Figures 7-8, a respiratory mask 20 according to the present invention comprises a mask body 21 forming a shell defining an internal respiratory chamber 22 fed with respiratory gas via a gas inlet 23, i.e. an opening arranged in the wall of the mask body 21. The gas inlet 23 of the mask body 21 has, for instance, a diameter of between 1 and 3 cm, and is located at the center of the front portion of the mask body 21.

The single-lip cushion 1 according to the present invention is fixed to the mask body 21 by means of a connecting border 9 cooperating with the end border of the mask body 21. The single-lip cushion 1 can be detached from the mask body 21. Further details about the single-lip cushion 1 are given below.

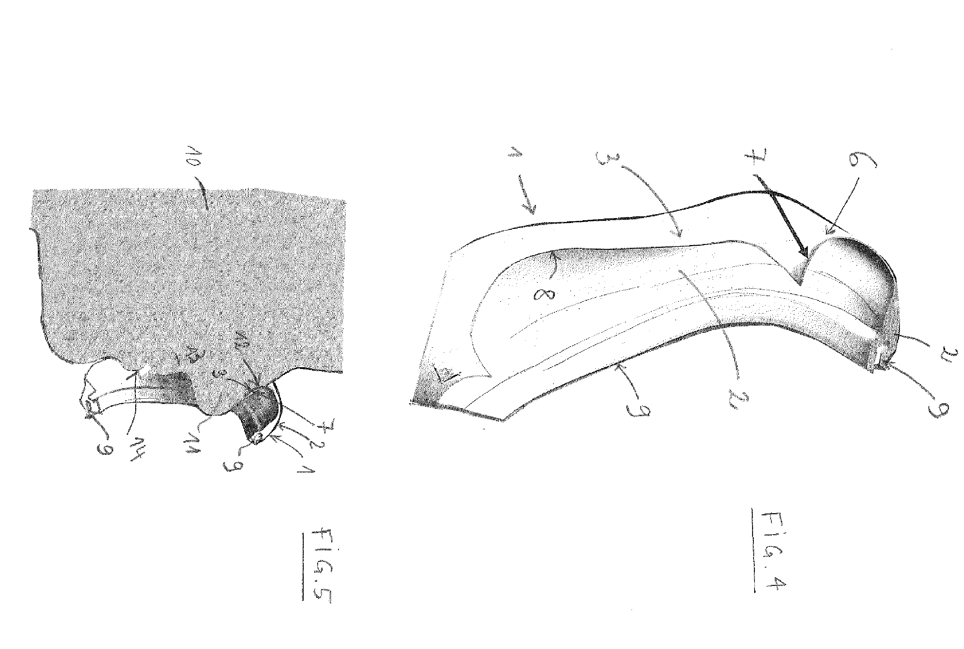

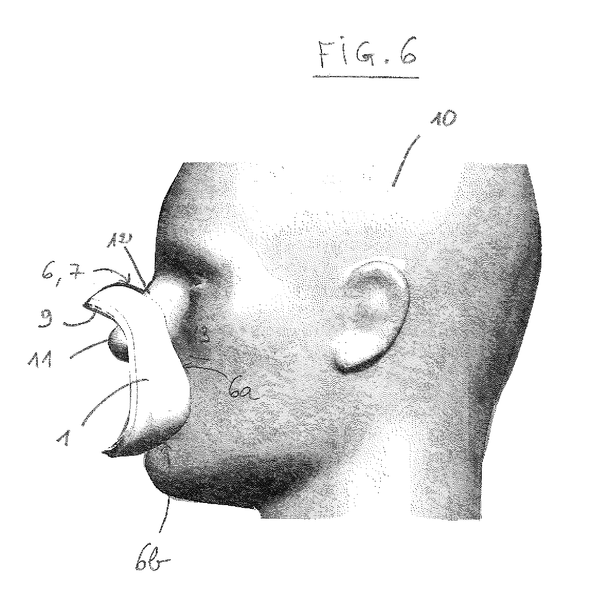

In the present embodiment, the respiratory mask 20 is a facial mask, and hence the cushion 1 is sized, i.e. dimensioned, to cover both the nose 11 and the mouth 14 of the patient 10, when used, as illustrated in Figures 5-6.

As shown in Figures 7-8, the mask body 21 comprises several arms 24, 26, namely two lateral arms 24 each projecting laterally on each side, i.e. right and left sides, of the mask body 21, and a holding arm 26 projecting upwardly from the mask body 21.

The two lateral arms 24 and the holding arm 26 that are integrally attached to the peripheral wall or surface of the mask body 1, preferably molded in one piece.

The arms have elongated shapes, for instance band or ribbon shapes, but other shapes are also possible. Nevertheless, arms 24, 26 and mask body 21 can also be made from several pieces or sub-units that are subsequently assembled together and made integral by means of any available fixing technique; for instance they can be glued, thermo-welded or similarly affixed to one another.

The arms 24, 26 comprise a free end comprising connecting structures 25, 27, i.e. strap-fixing systems, for fixing thereto a headgear, especially the straps of a headgear. A headgear is typically used for maintaining and securing the mask 1 in a desired position on the head of the user 10, i.e. the head of the patient. The straps of the headgear are typically made of polymer material or fabric material, or both. Here, the strap-fixing systems comprise slots 28 and hooks 25 as shown in Figures 7-8.

The mask body, the holding arm and of the two lateral arms are preferably made of a rigid material, like a polymer material.

Preferably, the arms 24, 26 are made of a flexible polymeric material so as to be slightly bendable toward the user's face under the forces applied by the headgear, when the latter is adjusted to the morphology of the user's head.

Examples of polymer materials that can be used are polycarbonate (PC), polypropylene (PP) or nylon.

Typically, the holding arm 27 has a length of about 2 and 10 cm, whereas each lateral arm 24 has a length in the range of about 3 to 8 cm.

Preferably, the holding arm 26 that projects upwardly from the mask body 21 also comprises a forehead support (not shown), which can comprise one or several frontal pads that come into contact with the forehead of the user 10, when the mask 20 is positioned on the user's face. The frontal pads act as bumpers and/or pillows that improve the comfort for the user 10 that wears the mask 20.

Further, the respiratory chamber 22 of the mask body 21 is fed with respiratory gas through the inlet orifice 23 of the main body 1, said gas being fed to the inlet orifice 23 by a hollow connector fixed to the mask body 21, such as a known rotatable curved gas connector (not shown).

Furthermore, as already mentioned, the mask 20 of the present invention also comprises an improved flexible cushion 1 that is fixed to the main body 21.

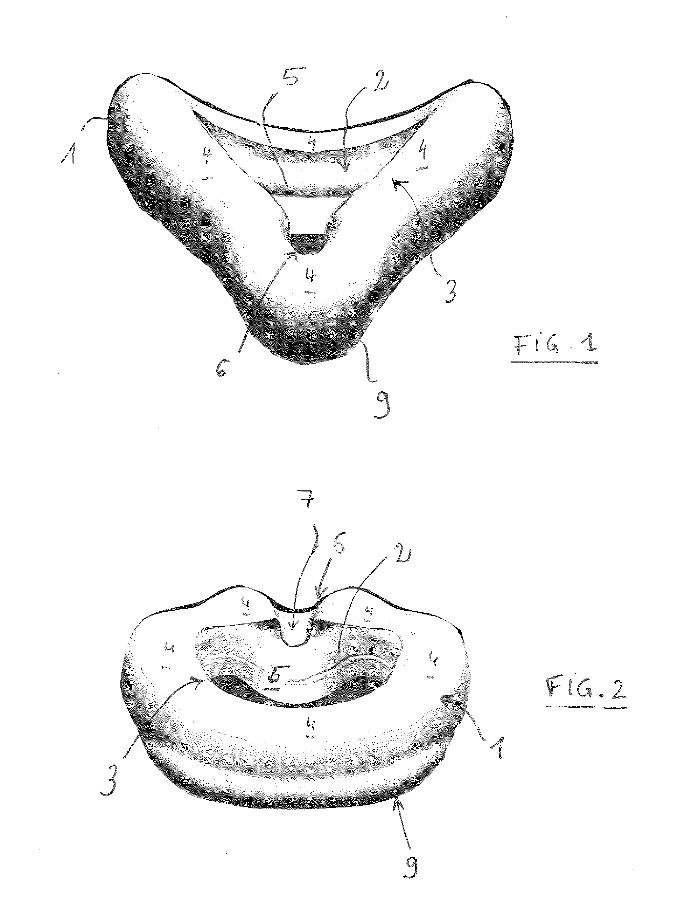

As better shown in Figures 1-6, the flexible cushion 1 is a tridimensional structure comprising a frame 2 having a triangular-shape and defining a nose aperture 5 adapted, i.e. conformed and sized, for receiving at least part of the patient's nose 11, when the patient 10 wears the mask 20 equipped with cushion 1, so that the patient 10 can breathe the respiratory gas contained in the respiratory chamber 22 of the mask body 21.

In order to provide efficient gas tightness (i.e. seal) and/or increased comfort for the patient, the nose aperture 5 of the cushion 1 is delimited by a single flexible membrane 3 that comes into contact with the patient's face and matches his/her facial morphology. The free border of the membrane 3 delimits the central aperture 5.

In use, the efficient gas tightness is, in particular, obtained thanks to the specific shape of the outer surface 4 of the membrane 3 that forms a convex sealing surface, i.e. an outer round-like structure, to the user's face 13, when the single-lip cushion 1 is positioned on the face 13 of the user 10 as shown in Figure 1-3.

In other words, the single membrane 3 constitutes a kind of soft flexible skirt delimiting the nose aperture 5 of the cushion 1 and ensuring gas tightness.

The single-lip cushion 1 of the invention comprises only one single membrane 3 (i.e. a unique membrane), namely no rim or no second membrane that might have been arranged underneath as advocated in some prior art documents.

The single membrane 3 or single lip is attached to the frame 2, preferably molded in one piece with said frame 2. Preferably, the flexible membrane 3 is fixed all along the perimeter of the frame 2 and projects away from said frame 2 as shown in Figure 4, namely the flexible membrane 3 is arranged in the continuity of the wall forming the triangular frame 2.

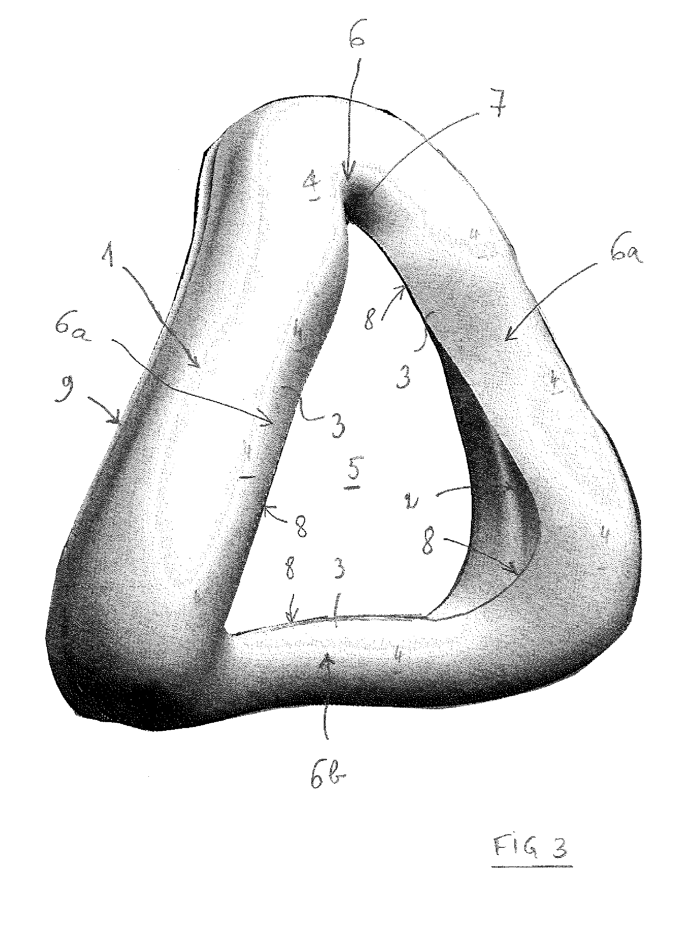

Further, as shown in Figure 3, the cushion 10 has a generally triangular, trapezoidal or saddle shape so as to match the contours of the patient's face.

When the facial mask 20 of Figures 7-8 is worn by the patient 10, i.e. the cushion 1 receives the patient's nose 11, while said patient 10 introduces his/her nose 11 through the nose aperture 5 of the cushion 1 and then breathes the gas present in the respiratory chamber 22 of the mask body 21. The membrane 3 of the cushion is then in contact with the nasal bridge area 6 of the nose 11 of the user 10 (area at the junction of the bone and cartilage of the nose, or slightly above, i.e. between said junction and the basis of the nose between the eyes), the chin area (area under the mouth, i.e. between the lower lip and the chin), and the two opposite lateral regions of the nose 11 of the patient (i.e. right and left flange regions of the nose), also called cheek regions. This is illustrated in Figures 5-6.

The cushion 1, including membrane 3 and frame 2, is preferably made of a flexible and soft material, such as silicone or the like.

In other words, the single-lip cushion 1 of the present invention comprises a frame 2 having a generally-triangular shape and a single flexible membrane 3 integrally fixed to the frame 2.

One important feature of the present invention concerns the flexible membrane 3 that comprises a nasal bridge region 6, as shown in Figures 1-4, that comes into contact with the nasal bridge area 12 of the user's nose 11, when the cushion 1 is positioned on the face 13 of the user 10 (cf. Fig. 5-6), which nasal bridge region 6 of the flexible membrane 3 is pre-shaped so as to form a cradle 7 that is inwardly-oriented and sized for accommodating at least the nasal bridge area 12 of the nose 11 of the user 10.

Indeed, thanks to the presence of said cradle 7 arranged in the nasal bridge region 6 of the membrane 3 of the cushion 1, the sealing features of the cushion 1 are greatly improved as the nasal bridge area of the user's nose spouses, i.e. better fits with, the walls of said cradle 7.

Preferably, the inwardly-oriented cradle 7 has a curved section, such as a "U"-shape section as visible in Figure 2.

Actually, the cradle 7 arranged in the nasal bridge region 6 of the membrane 2 forms a lodging or recess having gutter-like structure or similar that well-accommodates the nasal bridge area 12 of the patient's nose 11.

The inwardly-oriented cradle 7 arranged in the flexible membrane 3 is preferably obtained by molding while fabricating the cushion 1, including membrane 2 and frame 2.

Preferably, the thickness of the flexible membrane 3 is less than the thickness of the frame 2 so that the membrane 3 is more flexible than the frame 2.

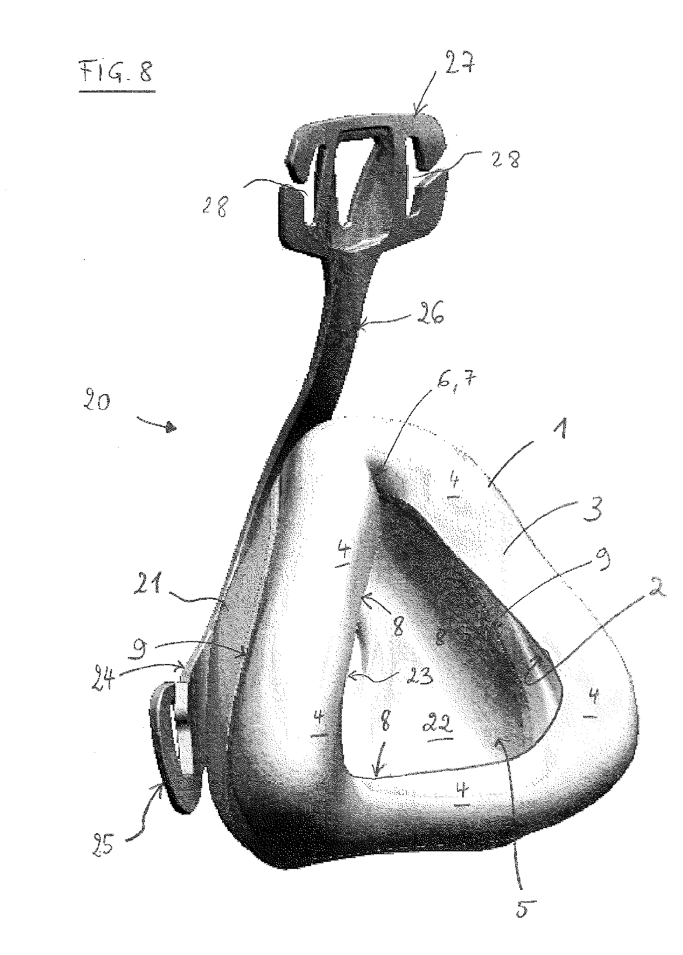

As shown in Figures 3 and 8, the central aperture 5 of the flexible membrane 3 has a substantially triangular or trapezoidal shape, which corresponds more or less to the shape of a human nose 11.

As above mentioned, the cushion 1 is fixed to the mask body 21 by means of a connecting system 9, such as male/female connections, such as grooves and walls arranged, on the one hand, on the cushion 1 and, on the other hand, on the mask body 21, and which are configured or shaped so as to cooperate and perfectly fit together, thereby maintaining the cushion1 attached to the mask body 21, in a detachable way so that the cushion 1 can be easily detached and replaced, after use.

Generally speaking, the respiratory mask 20 comprising the improved cushion 1 of the present invention, such as a facial mask 20, is easy to position and to secure on the patient's face, and ensures good gas tightness (i.e. seal) thereby improving the overall comfort of use for the patient 10.

The respiratory mask 20 equipped with an improved cushion 1 of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

In such a method for treatment, a respiratory gas, such as air or air enriched with oxygen, id delivered to a patient 10 by means of the respiratory mask 20 equipped with an improved cushion 1 of the present invention.

The respiratory gas is provided by a source of respiratory gas, typically a medical respiratory apparatus or ventilator that delivers the gas to the mask 20 via a (or more) gas conduct, such as flexible hose, and suitable gas connectors.

## Claims

1. Single-lip cushion (1) for a respiratory mask comprising a frame (2) having a generally-triangular shape and a single flexible membrane (3) integrally fixed to the frame (2), and wherein the flexible membrane (3) comprises a central aperture (5) for receiving at least the nose (11) of a user (10), and a nasal bridge region (6) that comes into contact with the nasal bridge area (12) of the user's nose (11), when the cushion is positioned on the face (13) ofthe user (10),
**characterized in that** the nasal bridge region (6) of the flexible membrane (3) is pre-shaped so as to form a cradle (7) that is inwardly-oriented and sized for accommodating at least the nasal bridge area (12) ofthe nose (11) of the user (10).

2. Single-lip cushion according to the preceding Claim, **characterized in that** the frame (2) and the flexible membrane (3) are made of a resilient material, preferably silicone.

3. Single-lip cushion according to any one of the preceding Claims, **characterized in that** the flexible membrane (3) further comprises an outer surface (4) forming a convex sealing surface to the user's face (13), when the cushion is positioned on the face (13) of the user (10).

4. Single-lip cushion according to any one of the preceding Claims, **characterized in that** the inwardly-oriented cradle (7) has a curved section.

5. Single-lip cushion according to any one of the preceding Claims, **characterized in that** the inwardly-oriented cradle (7) has a "U"-shape section.

6. Single-lip cushion according to any one of the preceding Claims, **characterized in that** the inwardly-oriented cradle (7) arranged in the flexible membrane (3) is obtained by molding.

7. Single-lip cushion according to any one of the preceding Claims, **characterized in that** the flexible membrane (3) is fixed all along the frame (2) and projects away from said frame (2), preferably the flexible membrane (3) is fixed along the perimeter of the frame (2).

8. Single-lip cushion according to any one of the preceding Claims, **characterized in that** the flexible membrane (3) and the frame (2) are molded in one-piece.

9. Single-lip cushion according to any one of the preceding Claims, **characterized in that** the frame (2) further comprises a connecting border (9) for fixing the cushion (1) to a respiratory mask body (21).

10. Single-lip cushion according to any one of the preceding Claims, **characterized in that** the thickness of the flexible membrane (3) is less than the thickness of the frame (2).

11. Single-lip cushion according to any one of the preceding Claims, **characterized in that** the central aperture (5) of the flexible membrane (3) has a substantially triangular or trapezoidal shape.

12. Respiratory mask (20) comprising a single-lip cushion (1) according to any one of the preceding Claims.

13. Respiratory mask according to Claim 12, **characterized in that** the mask is a facial mask, wherein the cushion (1) is sized to cover the nose (11) and the mouth (14) of the patient (10), when used.

14. Respiratory mask according to any one of Claims 12 to 13, **characterized in that** the mask body (21) comprises several arms (24, 26), preferably a headgear is fixed to the arms (24, 26) of the mask body (21).

15. Medical ventilation assembly comprising a medical ventilator and a respiratory mask according to claims 12-14, fluidly connected to the medical ventilator by means of at least a gas conduct.
